# EUROPEAN PATENT APPLICATION

(11) **EP 3 797 841 A1**
(43) Date of publication of application: **31.03.2021**
(21) Application number: 20198543.9
(22) Date of filing: 25.09.2020
(51) Int. Cl.: A63B 21/00, A63B 69/00, A61F 5/01

(54) **AN ORTHOPAEDIC ORTHOSIS FOR CARRYING OUT PRONOSUPINATION EXERCISES WITH ADJUSTABLE RANGE OF MOTION**

(30) Priority: 26.09.2019 IT 201900017342
(71) Applicant: F.G.P. S.R.L., 37062 Villafranca Di Verona (Verona) (IT)
(72) Inventor: FERRIGOLO, Moreno, 37062 Villafranca di Verona (IT); TURRINI, Alberto, 37062 Villafranca di Verona (IT)
(74) Representative: Sandri, Sandro

(57) **Abstract**

An orthopaedic orthosis for pronosupination of joints, comprising a handle (11) associated with a curved frame arm (12) which terminates with a bar (13) connected to a base wrapped around at least a part of a limb of a user by means of straps for harnessing the orthosis to the user's body. Said handle (11) is associated with said frame arm (12) by the interposition of a pivot (14) designed for adjustable rotation of the handle (11) relative to said curved frame arm (12), for the purpose of performing pronosupination exercises, and further comprising a curved connector (15) which connects the handle (11) to a first disk (16) having a central pin (17) that is part of the pivot (14), and wherein said pivot (14) further comprises a second disk (20) having a central hole (21) associated with said pin (17) so that the curved frame arm (12) remains constrained, with the possibility of rotating, to the first disk (16) and to the handle (11). Said second disk (20) comprises, along the outer circular sector thereof, a plurality of holes (22), disposed in a radial direction, which are each located at a same angular inter-distance from each other, and there is present a pair of plugs (24) which are introduced into two respective holes (22) of said plurality of holes at the beginning and at the end of the angular travel of the handle (11).

## Description

### FIELD OF APPLICATION

The present invention relates to an orthosis applicable on limbs, typically upper limbs, such as the forearm, and comprising a means for adjusting an angular travel (ROM - range of motion) applied by means of insertable/extractable inserts, for the purpose of performing pronosupination exercises.

The orthosis according to the invention enables the adjustment of a rehabilitation device with an angular rotation that is limited in a range defined by two inserts.

It is envisaged that said orthosis, destined for a use mainly intended for the forearm, is provided with a handle or another device which connects the limb to the ROM system of the orthosis and is endowed with a projection or tooth which, upon rotating, becomes locked against two limit stops.

In the situation where the stops are positioned on the two sides of the tooth, the handle remains locked in its current position.

The present invention has application in the medical and orthopaedic industries and in particular in the production of braces in general, as well as of protheses and braces mainly utilised in conservative, post-traumatic, rehabilitative and postoperative therapy.

### PRIOR ART

It is known that individuals having orthopaedic problems of the knee joints, but similarly of other joints as well, such as the ankle or elbow joints, above all in the event of injuries or as a consequence of a previous surgical intervention, need to use an orthopaedic brace, or orthosis, designed to ensure or control the hinge constraint function between the arm and the forearm, or between the femur and the tibia or other lever pivot points of joints, while withstanding stresses that would otherwise be damaging for the joint itself.

One of the functions of an orthosis is as an aid to functional rehabilitation or re-education, where the orthosis can be used to perform, for example during convalescence, rehabilitation exercises in order to restore joint functions that have been limited by surgical interventions or injuries.

An orthosis usually consists of a rigid or soft frame, wrapped around the upper or lower limb by means of special straps, and adapted to ensure adequate harnessing of the joint in order to prevent the occurrence of stresses during walking or during movement of the arms of the injured and/or convalescent individual.

According to the prior art, the frame of an orthosis for joints, for example in the case of arm braces designed, for example, for rehabilitation of the wrist, comprises a means of constraint to the arm and forearm, and a connection section of said means consisting of a pivot placed at the knee or elbow or wrist.

Again in the case of the forearm, the movement of pronation and supination of the wrist is made possible by the presence of two trochoid joints between the radius and ulna, a proximal one (elbow) and a distal one (close to the wrist).

A prior art document in the sector of rehabilitative orthoses for pronosupination is for example WO2013040350, which regards an orthosis for the supination and/or pronation of a forearm of a user, the orthosis comprising a support frame provided with two ends, the first of which is intended to receive a means for the retention and rotation of the forearm at the wrist, whereas the second is connected to a support disposed on an axis orthogonal to the frame, the support comprising a means for retaining the arm.

The means for the retention and rotation of the forearm are represented by a circular support placed on the rotation axis of the forearm and suitable for engaging at the wrist. Said support is rotatable, by means of specific gears, relative to the frame on the rotation axis of the forearm, and is configured to engage the wrist and the forearm of the wearer, enabling passive mechanised pronosupination.

However, it has been found that this solution is highly complex and consequently expensive for whoever produces it as well as for whoever purchases it and, moreover, it makes it very difficult to regulate the movements of pronosupination of the wrist in an angular range that must be varied and progressively increased to enable an increase in the operating angle during the rehabilitation exercises for the cycle of recovery of the wrist's movement.

Consequently, to date the problem of active pronosupination with an adjustable angle remains unsolved, since the braces known today are complex and costly, unsuitable for aerobic rehabilitation exercises and therefore difficult to introduce into the market.

### DESCRIPTION OF THE INVENTION

The present invention aims to provide an orthosis enabling one to perform pronosupination exercises of joints such as the wrist, but also the ankle, knee, elbow, shoulder or other joints, and which is capable of eliminating or at least reducing the drawbacks and limits revealed in traditional braces.

According to the invention it is envisaged that the orthosis is provided with a means that enables the adjustment of an angular ROM applied by means of insertable/extractable inserts.

This is achieved through a means for adjusting an angular ROM in order to perform pronosupination exercises, whose features are described in the main claim.

The dependent claims of the solution in question outline advantageous embodiments of the invention.

The main advantages of this solution regard first of all the fact that said means enabling the adjustment of an angular ROM is represented by insertable/extractable inserts which are disposed at the minimum and maximum angle provided for in a given rehabilitation exercise.

In particular, again in the case of the wrist, it is envisaged to use a handle gripped by the patient's hand and associated with a frame wrapped around the upper limb by means of special straps so as to ensure adequate harnessing, wherein said handle is associated with said frame by the interposition of a pivot that enables the adjustable rotation of the handle during the pronosupination exercises.

The adjustment of the ROM or angular travel is determined by different positioning of said inserts along the crown of said pivot, the inserts acting as stops for a tooth projecting from the handle.

### ILLUSTRATION OF THE DRAWINGS

Other features and advantages of the invention will become apparent upon reading the following description of one embodiment of the invention, provided by way of nonlimiting example with the aid of the drawings illustrated in the appended figures, in which:
- figure 1 represents an overall schematic axonometric view of a brace or orthosis according to the invention, designed for performing pronosupination exercises, with the components in an exploded view;
- figure 2 is an overall schematic side view of the orthosis according to the invention with the components assembled;
- Figure 3 illustrates a detailed sectional view of the pivot of the orthosis according to the line B-B in figure 2;
- Figures 4 to 6 represent schematic views illustrating the system of angular adjustment of the orthosis according to the invention according to a first configuration;
- Figures 7 to 9 show schematic views illustrating the system of angular adjustment of the orthosis according to the invention in a second configuration.

### DESCRIPTION OF AN EMBODIMENT OF THE INVENTION

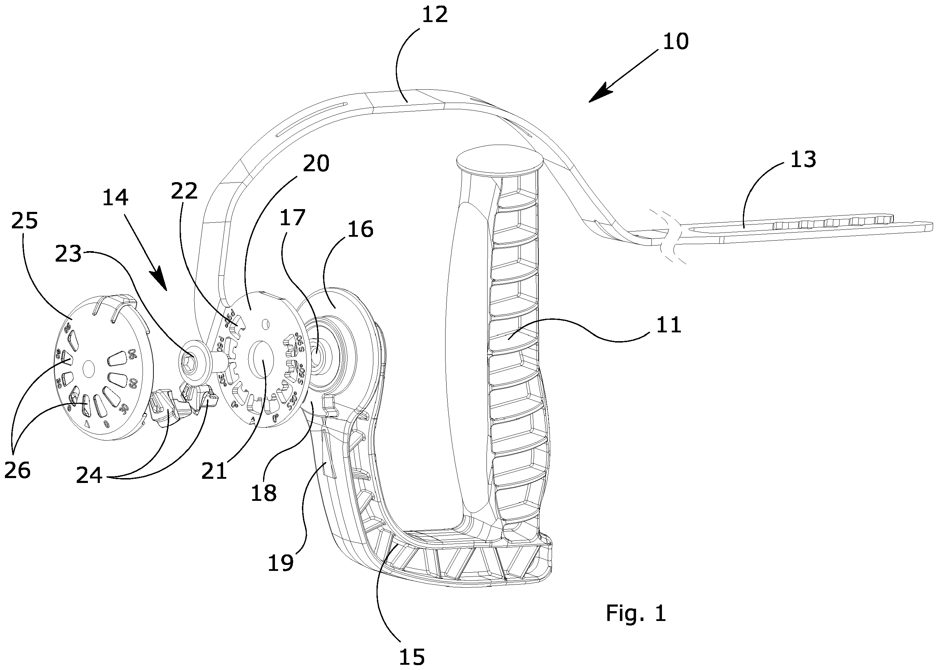

Making reference initially to figure 1, the reference number 10 denotes in its entirety an orthosis which enables one to perform pronosupination exercises of joints such as the wrist, but also the ankle, knee, elbow, shoulder or other joints.

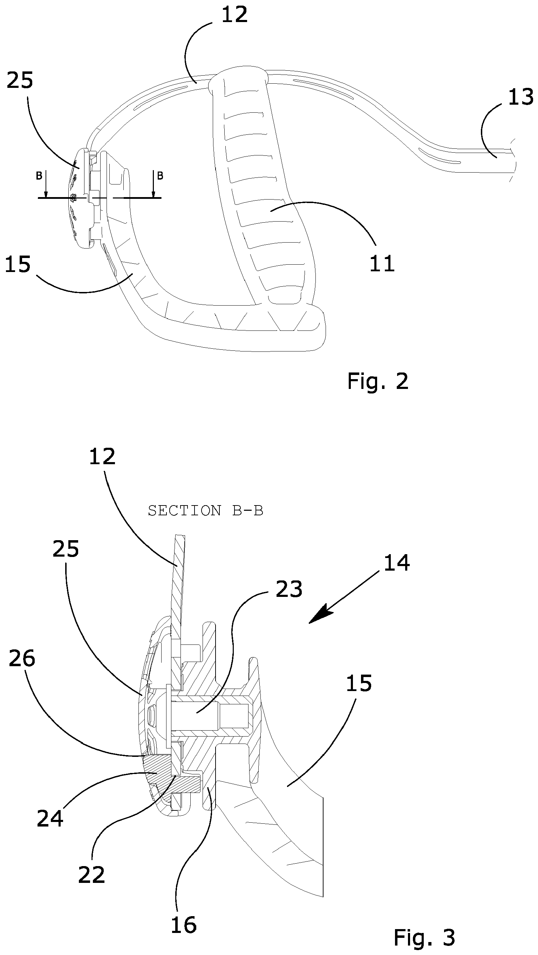

According to the embodiment represented in figures 1 to 9, the orthosis comprises a handle 11 associated with a curved frame arm 12 which terminates with a bar 13 connected to a base wrapped around the upper limb by means of special straps so as to ensure adequate harnessing.

More precisely, said handle 11 is associated with said frame arm 12 by the interposition of a pivot 14 which enables adjustable rotation of the handle itself during the pronosupination exercises.

More in particular, the handle 11 is connected on one side by means of a curved connector 15, bearing at the end thereof a first disk 16 comprising a central pin 17 and a tooth 18 located in the circular sector thereof in proximity to a reference notch 19 located along said curved connector 15.

Said frame arm 12 in turn terminates at the end thereof with a second disk 20 provided with a central hole 21 associable with said pin 17, so that the arm 12 remains constrained, with the possibility of rotating, to the first disk 16 of the handle 11.

The second disk 20 comprises, along the outer circular sector thereof, a plurality of holes 22, disposed in a radial direction, which are each located at a same angular inter-distance from each other.

Furthermore, the second disk 20 is keyed, with the possibility of rotating, onto the pin 17 of the first disk 16 and held in this position by a retaining means 23, for example of a screw type or the like.

When the bar 13 is secured with its straps for example to the forearm, this arrangement allows the possibility of gripping the handle 11 and carrying out pronosupination movements according to a complete, i.e. totally free, angle, even of 180° or more.

According to one feature, the holes 22 can be intercepted by a pair of plugs 24, or stops, which are introduced at the beginning and at the end of the angular travel of the handle 11, the range of travel being established on the basis of the different needs in terms of the movement to be carried out at a given stage of rehabilitation of the wrist or other parts of the body to be rehabilitated.

The plugs 24 are introduced into the holes 22 in a direction parallel to the axis of reciprocal rotation of the disks 16 and 20.

The retention of the plugs 24 in their seat is ensured by a cover 25 endowed with openings 26 from which a part of the plugs 24, or stops, projects, enabling the immediate detection of their position.

The cover 25 is retained on the second disk 20 by means of an elastic snap-fit means or the like.

The use of the orthosis thus comprises a step of adjusting the plugs 24, which provides for the positioning thereof, after removal of the cover 25, according to the established angular travel, in two of the holes 22 located on the disk 20, in such a way as to project inwards towards the disk 16 in order to be intercepted by the tooth 18.

The position of the plugs 24, or stops, determines the maximum and minimum angular travel that can be carried out by the handle 11, based on the pronosupination exercise to be performed.

Once the plugs 24 have been introduced, it is sufficient to close off the pivot with the cover 25, which allows the position of the plugs 24 to be viewed by means of the openings 26 provided.

Based on how the plugs 24 are positioned, the angular rotation of the handle can be limited from -90° (90° in pronation) to +90° (90° in supination).

In the embodiment represented, the holes 22 are spaced apart from each other by 30°.

In this manner (in the case of the illustrated examples), the stop plugs can be inserted at -90°, -60°, -30°, 0°, +30°, +60°, +90°.

Furthermore, in this range it is also possible to set the locking of the handle 11 by inserting the two plugs 24 adjacent to the tooth 18 of the handle itself.

The adjustment of the ROM or angular travel is thus determined by the different positioning of said plugs 24 along the crown of said pivot, the plugs acting as a stop for the tooth 18 projecting from the handle 11 and positioned on the first disk 16 in proximity to the reference notch 19.

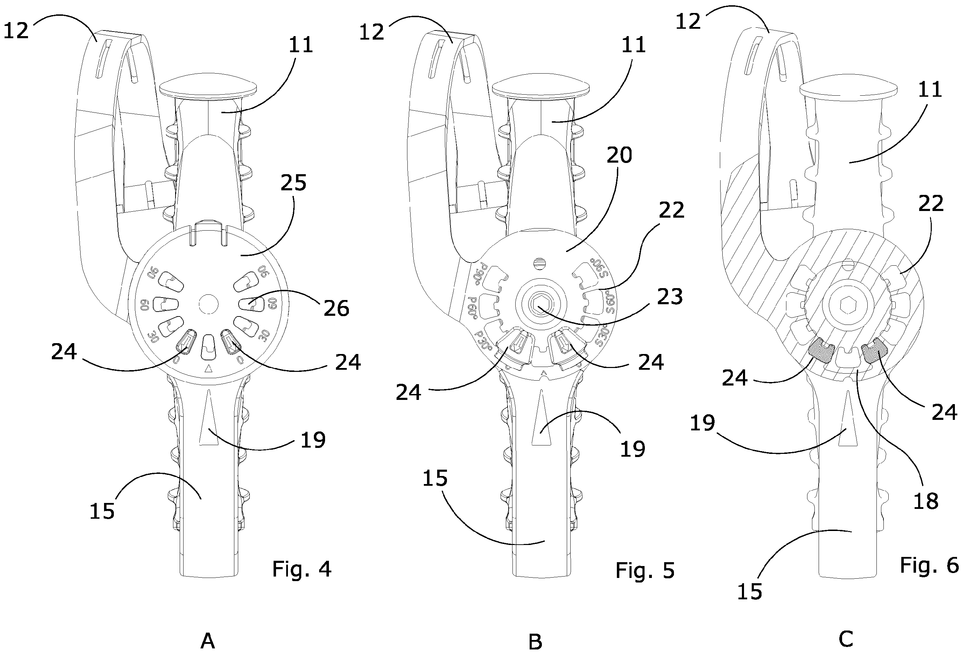

In figures 4 to 6, the pivot 14 is represented in the locking position of the plugs 24 at 0°. In these three pictures one can see different levels of the handle 11 locked at 0° with the two plugs 24 positioned at the two sides of the tooth 18.

In figure A, the fastening cover can be seen in the closed position, in figure B the two plugs can be seen, and in figure C a section with the plugs 24 shaded in (the part acting as a stop) and the outline of the tooth 18 of the handle can be seen.

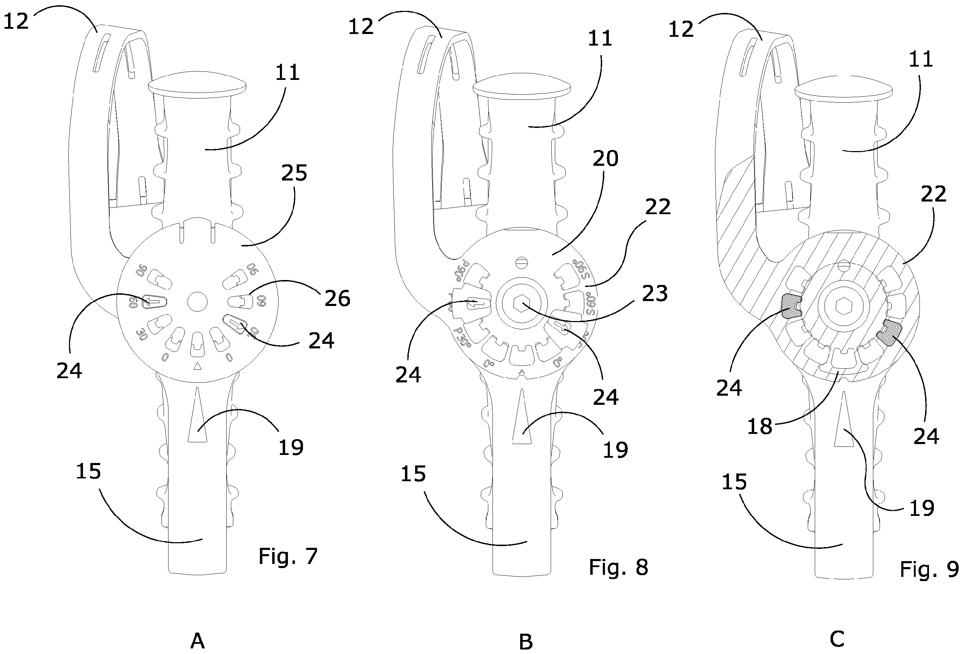

Figures 7 to 9 illustrate the locking position that the plugs 24 take on at 60° of pronation and 30° of supination. In this case, the handle 11 is free to move between 60° of pronation and 30° of supination.

In figure A, the fastening cover can be seen in the closed position, in figure B the two plugs can be seen, and in figure C a section with the plugs 24 shaded in (the part acting as a stop) and the outline of the tooth 18 of the handle can be seen (in the 0° position).

The invention has previously been described with reference to a preferred embodiment thereof. However, it is clear that the invention is susceptible of numerous variants falling within the scope thereof, within the framework of technical equivalences.

## Claims

1. An orthopaedic orthosis for performing pronosupination exercises of joints, comprising a handle (11) associated with a curved frame arm (12) which terminates with a bar (13) connected to a base wrapped around at least a part of a limb of a user by means of straps for harnessing the orthosis to the user's body, wherein said handle (11) is associated with said frame arm (12) by the interposition of a pivot (14) designed for adjustable rotation of the handle (11) relative to said curved frame arm (12), for the purpose of performing pronosupination exercises, further comprising a curved connector (15) which connects the handle (11) to a disk (16) having a central pin (17) that is part of the pivot (14) and a tooth (18) positioned in proximity to a reference notch (19) located along said curved connector (15), and wherein said pivot (14) further comprises a second disk (20) having a central hole (21) associated with said pin (17) so that the curved frame arm (12) remains constrained, with the possibility of rotating, to the first disk (16) and to the handle (11), **characterised in that** said second disk (20) comprises, along the outer circular sector thereof, a plurality of holes (22), disposed in a radial direction, which are each located at a same angular inter-distance from each other, and **in that** there is present a pair of plugs (24) which are introduced into two respective holes (22) of said plurality of holes at the beginning and at the end of the angular travel of the handle (11).

2. The orthopaedic orthosis according to claim 1, **characterised in that** said second disk (20) is keyed, with the possibility of rotating, onto the pin (17) of the first disk (16) and held in this position by a pin-type retaining means (23).

3. The orthopaedic orthosis according to claim 2, **characterised in that** said plugs (24) are introduced into the holes (22) in a direction parallel to the axis of reciprocal rotation of said first and second disks (16, 20).

4. The orthopaedic orthosis according to one of the preceding claims, **characterised in that** the retention of the plugs (24) in their seat is obtained by means of a cover (25) of said pivot (14) endowed with openings (26) from which part of the plugs (24) project, enabling an immediate detection of their position through said openings (26).

5. The orthopaedic orthosis according to claim 4, **characterised in that** said cover (25) is retained on the disk (20) by means of an elastic snap-fit means or the like.

6. The orthopaedic orthosis according to claim 5, **characterised in that** the adjustment of the plugs (24) in order to establish the angular travel of the pivot (14) provides for the positioning thereof, after removal of the small cover (25), in two of the holes (22) located on the second disk (20), in such a way as to project inwards towards the disk (16) in order to be intercepted by said tooth (18), which determines the angular travel of the handle (11) between two set limits.
